# EUROPEAN PATENT SPECIFICATION

(11) **EP 1 437 483 B1**
(45) Date of publication and mention of the grant of the patent: **21.04.2010**
(21) Application number: 04250140.3
(22) Date of filing: 13.01.2004
(51) Int. Cl.: F01D 9/02, F01D 5/18

(54) **Trailing edge cooling of a turbine airfoil**
Kühlung der Abströmkante einer Turbinenschaufel
Refroidissement du bord de fuite d'une aube de turbine

(30) Priority: 13.01.2003 US 340971
(43) Date of publication of application: 14.07.2004
(73) Proprietor: United Technologies Corporation, Hartford, CT 06101 (US)
(72) Inventor: Boyer, Jason W, Middletown, CT 06457 (US)
(74) Representative: Leckey, David Herbert

(56) References cited:
- EP-A- 0 034 961
- EP-A- 0 392 664
- EP-A- 1 333 154
- US-A- 3 420 502
- US-A- 5 243 759
- US-A- 5 503 529
- US-A1- 2002 197 161

## Description

This invention relates to turbomachinery, and more particularly to cooled turbine blades and vanes.

Trailing edge cooling is a common feature of turbine blades and vanes. In one common method of manufacture, the main passageways of a cooling network within the blade/vane airfoil are formed utilizing a sacrificial core during the blade/vane casting process as for example in US 2002/0197161. The airfoil surface may be provided with holes communicating with the network. Some or all of these holes may be drilled. In one method of manufacture, an array of trailing edge holes may be drilled parallel to each other and at an even pitch.

According to one aspect of the present invention, there is provided a method of manufacturing a turbine element as claimed in claim 1.

According to another aspect of the present invention, there is provided a turbine element as claimed in claim 5.

The internal cooling passageway network may be adapted to direct cooling gas within the trailing edge cavity to increase in temperature in a first direction parallel to the trailing edge. The spacing may substantially progressively decrease in that first direction. The trailing edge cavity may be an impingement cavity.

The details of one or more embodiments of the invention are set forth in the accompanying drawings and the description below. Other features and advantages of the invention will be apparent from the description and drawings, and from the claims.
FIG. 1 is a view of a turbine vane.
FIG. 2 is a partial sectional view of the vane of FIG. 1, taken along line 2-2.
FIG. 3 is a view of the vane of FIG. 2, taken along line 3-3.
FIG. 4 is a view of a vane/blade manufacturing apparatus.
FIG. 5 is an x-ray view of a turbine blade.

Like reference numbers and designations in the various drawings indicate like elements.

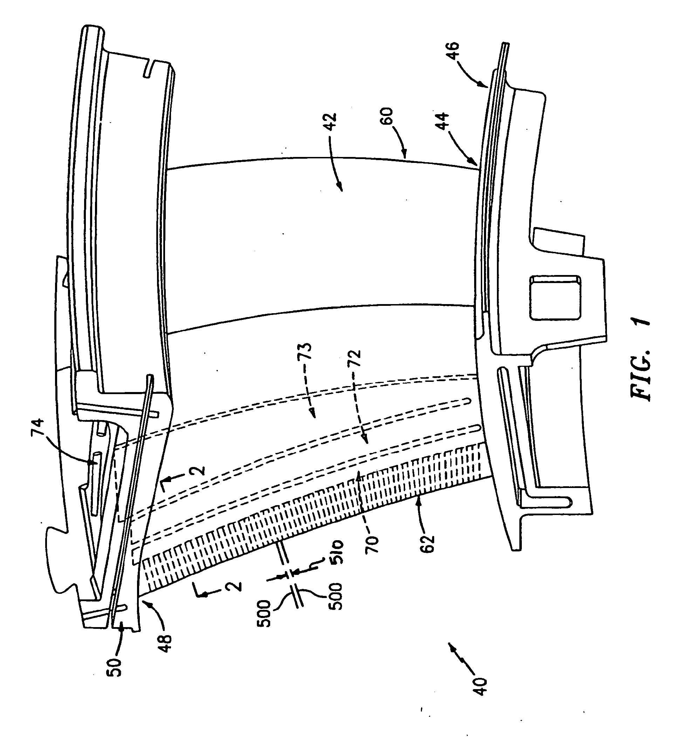

FIG. 1 shows a turbine vane 40 having an airfoil 42 extending along a length from a proximal root 44 at an inboard platform 46 to a distal end 48 at an outboard platform 50. A number of such vanes may be assembled side-by-side with their respective inboard and outboard platforms forming inboard and outboard rings bounding inboard and outboard portions of a flow path. In an exemplary embodiment, the vane is unitarily formed of a metal alloy.

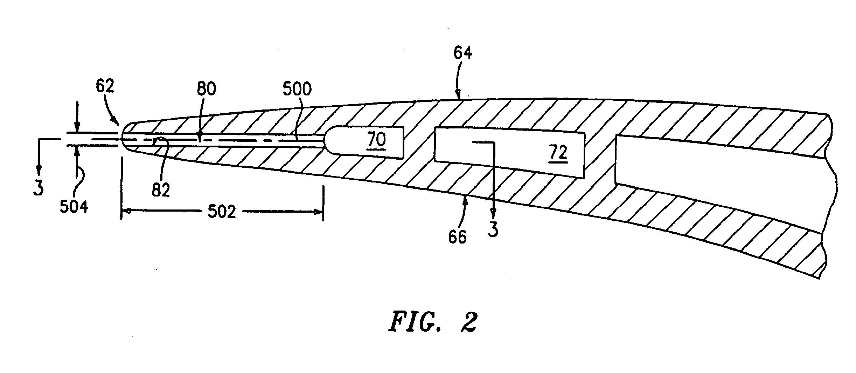

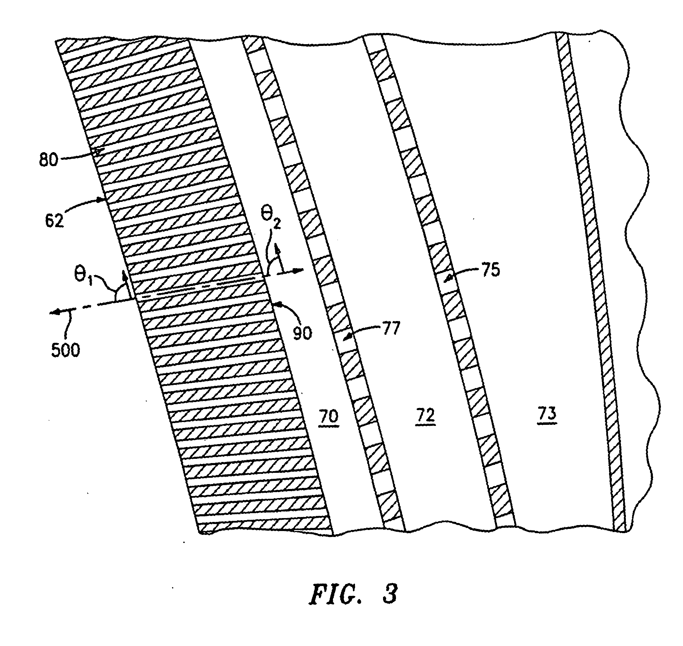

The airfoil extends from a leading edge 60 to a trailing edge 62. The leading and trailing edges separate pressure and suction sides or surfaces 64 and 66 (FIG. 2). For cooling the airfoil, the airfoil is provided with a cooling passageway network coupled to ports in one or both platforms. The exemplary passageway network includes a series of cavities extending generally lengthwise along the airfoil. An aftmost cavity is identified as a trailing edge cavity 70 extending generally parallel to the trailing edge. A penultimate cavity 72 is located ahead of the trailing edge cavity 70. The cavities may be joined at one or both ends and/or locations along their lengths so as to permit flows from the penultimate cavity to the trailing edge cavity. In the illustrated embodiment, the cavities 70 and 72 are impingement cavities. The penultimate cavity 72 receives air from a supply cavity 73 through an array of apertures 75 (FIG. 3) in the wall separating the two. The supply cavity 73 receives air from a port 74 in the platform 50. Likewise, the trailing edge cavity 70 receives air from the penultimate cavity 72 via apertures 77 in the wall between the two. To the extent that the cooling air in the supply cavity 73 is heated as it progresses radially inward, the cooling air temperatures in the impingement cavities 70 and 72 will similarly increase in the radially inward direction.

The network may further include holes extending to the pressure and suction surfaces 64 and 66 for further cooling and insulating the surfaces from high external temperatures. Among these holes may be an array of trailing edge holes 80 extending between a location proximate the trailing edge and an aft extremity of the trailing edge impingement cavity 70. FIG. 2 shows one such hole having a surface 82 centered about an axis 500 and extending along a length 502. The exemplary hole has a circular section with a diameter 504.

FIG. 3 shows a portion of the array of trailing edge holes 80. The holes, or more precisely their axes 500, are shown as at an angle θ₁ relative to the trailing edge and at an angle θ₂ relative to the local aft extremity 90 of the trailing edge impingement cavity 70. To the extent that the trailing edge 62 and aft extremity 90 are parallel, the illustrated angles θ₁ and θ₂ will be identical to each other for a given hole 80. A pitch 510 of the holes is measured as the hole centerline spacing along the trailing edge.

As is explored in further detail below, the axes 500 of every hole 80 need not be parallel to each other. Similarly, the angles θ₁ and/or θ₂ of each hole 80 need not be the same, nor need be their diameters 504 and lengths 502. Structural integrity and manufacturing considerations may influence or dictate the separation of the trailing edge 62 from the aft extremity 90 of the cavity 70. It is advantageous that the holes 80 be short and narrow so as to maximize possible cooling close to the trailing edge. The narrowness (e.g., the diameter) is largely limited by ease of drilling. Subject to additional manufacturing and terminal considerations (discussed below) this minimization would be achieved by having the axes 500 as close as possible to mutually perpendicular to the trailing edge 62 and aft extremity 90.

Along the trailing edge, less cooling may be required per linear dimension along one portion of the trailing edge than along another. In the exemplary embodiment, with cooling air flowing generally radially inward in the supply cavity 73, the air passing through the impingement cavities 72 and 70 and through the holes 80 may be cooler near the end 48 than near the root 44 due to the shorter net flow path. As this cooler air is more effective for heat transfer, less volume of air per linear dimension need be passed through the holes 80 near the end 48 than near the root 44. Accordingly, to efficiently utilize the cooling air passing through the cavity 70, it may be advantageous that the hole spacing generally increase in an outboard direction along the trailing edge. Ignoring manufacturing considerations and terminal considerations, the change in spacing could well be continuous, with a slight change in spacing from each hole to the next in accordance with an appropriate cooling distribution.

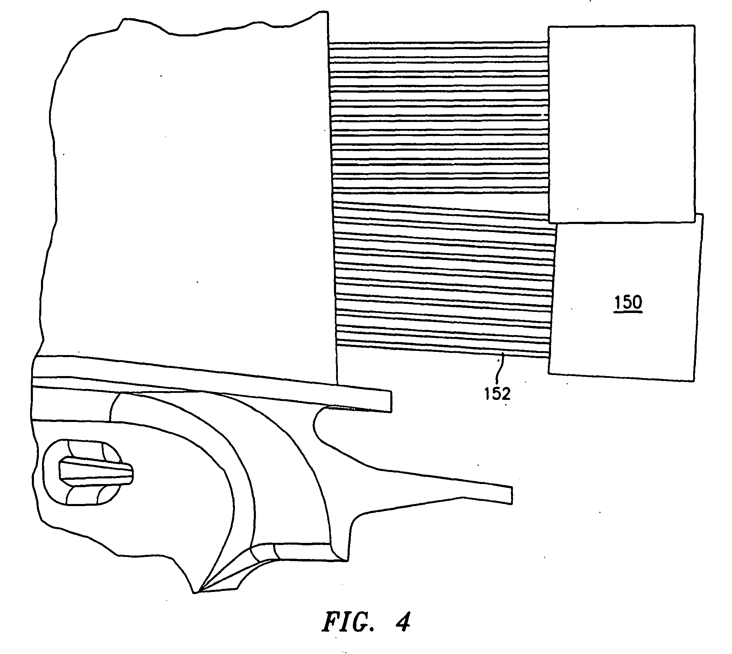

If, for example, the terminal end of the cavity 70 did not extend as far as the outboard end of the trailing edge, it might be desirable to slightly fan the holes near the outboard edge or otherwise enhance cooling. The inboard end of the trailing edge may also pose manufacturability problems due to interference with a drilling apparatus. If it is desired to drill the holes perpendicular to the trailing edge, the inboard platform may interfere with drilling of the inboardmost hole or holes along the trailing edge, thus, for a given drilling apparatus, a restriction to perpendicular holes might place the inboardmost hole too far outboard. Accordingly, for this hole such considerations may cause a reduction in the angle θ₁ below 90° so as to permit the hole to be sufficiently inboard. Also, access to the trailing cavity at inboard or outboard ends of the trailing edge may alter the angle θ₁ from that which might otherwise be desirable. Additionally, it may be desired to gang drill several holes at a time with a single drilling apparatus 150 (FIG. 4) having several bits 152 (10 bits shown). The use of such apparatus may restrict freedom in the spacing selection and in the hole orientation. One such apparatus might require the several gang-drilled holes to be parallel to each other, preventing independent selection of the angle θ₁ for each hole. Ease of constructing such apparatus might require that the several hole axes be evenly spaced from each other, thereby preventing independent selection of spacing for each hole. Alternatively, however, the spacing could change along a given apparatus with a characteristic spacing (e.g., a mean or median) of one apparatus differing from that of the next. With such apparatus, a continuous change in spacing may be achieved.

With this in mind, in one gang-driling example, a single drilling apparatus is utilized to drill a given number N (e.g., 5-15) of holes at a time. The apparatus may be used to drill a number M (e.g., 5 or more) of sets of such N holes with an exemplary total number of holes being between 40 and 200. The axes of each set could be nonparallel to the axes of the other sets, thus permitting the sets of holes to be relatively close to perpendicular to the trailing edge (again subject to departures due to terminal considerations), and according to the present invention the axes of at least two sets must be non-parallel to each other. In another example, different drilling apparatus 150 having different axis spacing may be utilized to drill the different sets of holes.

By way of example, the radial span of the trailing edge may be about 1.0-15 inches (25.4-381 mm) depending on the application. The hole diameters may be between about 0.01 inch (0.25 mm) and 0.15 inch (3.81 mm), more narrowly about 0.015-0.025 inch (0.381-0.635 mm). The hole length may be between 5-25 times the hole diameter. In an exemplary vane embodiment, the vane is dimensioned so that, when the ring is assembled, the root at the trailing edge is at a radius of about ten inches relative to the engine centerline. The outboard end of the trailing edge is at a radius of about 12.5 inches (317 mm). In the exemplary embodiment, the spacing starts at approximately 2.1 times the hole diameter near the inboard platform, remains generally the same until the middle of the length of the trailing edge and then increases to approximately 2.7 times the diameter toward the outboard platform. Thus one drilling apparatus with the smaller spacing may drill several groups of holes and then a second apparatus having the larger spacing may drill the remainder (which in the exemplary embodiment is a slightly smaller number of holes). In the exemplary embodiment, the hole length varies from approximately 14.5 times the hole diameter near the inboard platform to approximately 13.75 times the hole diameter near the outboard platform. In the exemplary embodiment, along about the inboardmost 10% of the trailing edge, the holes near the inboard platform are at a spacing larger than the 2.1 figure due to a reduced cooling need near the platform. Thus it can be seen that the progressive spacing may be over only a substantial portion of the trailing edge (e.g., 40-90% or, more narrowly, 50-80%).

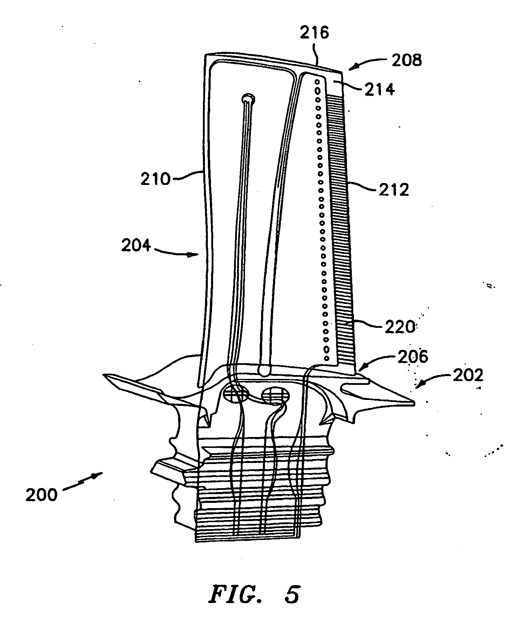

FIG. 5 shows a turbine blade 200 having a platform 202 and an airfoil 204 extending from a proximal root 206 at the platform to a distal end tip 208. The airfoil may have substantial similarities to the vane airfoil. In given turbine, and a number of such blades may be positioned with their platforms side-by-side to form a ring. Such blade rings may be interspersed with the vane rings, the inboard platforms of both forming a generally continuous inboard wall of the flow path through the turbine. The exemplary blade airfoil has leading and trailing edges 210 and 212 separating pressure and suction sides 214 and 216. The blade airfoil may have an array of trailing edge holes 220 similar to that of the vane airfoil.

One or more embodiments of the present invention have been described. Nevertheless, it will be understood that various modifications may be made without departing from the scope of the invention. For example, desired flow characteristics of the turbine may influence hole arrangement in view of available manufacturing techniques. This is particularly true in redesign or retrofit of existing turbines. Accordingly, other embodiments are within the scope of the following claims.

## Claims

1. A method of manufacture of a turbine element (40; 200) having:
a platform (46; 202); and
an airfoil (42; 204):
extending along a length from a proximal root (44; 206) at the platform (46; 202) to a distal end (48; 208);
having leading and trailing edges (60, 62; 210, 212) separating pressure and suction sides (64, 66; 214, 216); and
having a cooling passageway network including at least one trailing edge cavity (70) and a plurality of trailing edge holes (80) extending from the trailing edge (62; 212) to the trailing edge cavity (70), the method comprising:
casting a turbine element precursor;
machining simultaneously with one another a first group of said plurality of said holes (80); and
machining simultaneously with one another a second group of said plurality of holes (80), displaced from the first group of holes along the trailing edge (62; 212), having a characteristic spacing differing from a characteristic spacing from the first group;
wherein centerlines (500) of said first group of holes (80) are non-parallel to centerlines of said second group of holes (80).

2. The method of claim 1, wherein the at least one trailing edge cavity (70) is an impingement cavity.

3. The method of claim 1 or 2, wherein the centrelines of said first or second group of holes (80) are substantially perpendicular to the trailing edge (62; 212).

4. The method of claim 1, 2 or 3, wherein the machining of the first group is performed using a first apparatus (150) and the machining of the second group is performed using a second apparatus (150), and the method further comprises simultaneously machining a third group of said plurality of trailing edge holes (80) using said first apparatus.

5. A turbine element (40; 200) comprising:
an airfoil (42; 204) having:
first and second ends (44, 48; 206, 208);
leading and trailing edges (60, 62; 210, 212); and
an internal cooling passageway network including at least one trailing edge cavity (70) and a plurality of trailing edge holes (80) extending from the trailing edge (62; 212) to the trailing edge cavity (70),
wherein the trailing edge holes are arrayed at a spacing which progressively changes along a portion of said trailing edge (62; 212) from the first end (44; 206) toward the second end (48; 208);
**characterised in that** there are several groups of said trailing edge holes (80), the holes in each said group having centrelines (500) parallel to each other, and the centrelines (500) of holes in at least two of said groups being non-parallel to each other.

6. The element of claim 5, wherein the centrelines (500) of holes in at least one of said groups are substantially perpendicular to the trailing edge.

7. The element of claim 5 or 6, wherein the spacing increases substantially continuously.

8. The element of claim 5 or 6, wherein the spacing increases substantially stepwise over groups of said trailing edge holes (80).

9. The element of any of claims 5 to 8, being a blade (200) wherein said second end is a free tip (208) and the first end is a root end (206).

10. The element of any of claims 5 to 8, being a vane (40) wherein said first and second ends (44, 48) respectively join inboard and outboard platforms (46, 50) unitarily formed with the airfoil (44).

11. The element of any of claims 5 to 10, wherein the trailing edge holes (80) have diameters between 0.381 - 0.635mm (0.015 and 0.025 inch).

12. The element of any of claims 5 to 11, wherein the trailing edge cavity (70) is an impingement cavity.

13. The element of any of claims 5 to 12, wherein:
the network is adapted to direct cooling gas within the trailing edge cavity (70) to increase in temperature in a first direction parallel to the trailing edge (62; 212); and
the spacing substantially progressively decreases in the first direction.

14. The element of any of claims 5 to 13, wherein:
the network is adapted to direct cooling gas within the trailing edge cavity (70) to increase in temperature in a first direction parallel to the trailing edge (62; 212);
the portion is at least 50% of a length of the trailing edge (62; 212); and
the spacing continuously progressively decreases in the first direction.

## Patentansprüche

1. Herstellungsverfahren für ein Turbinenelement (40; 200) mit
einer Plattform (46; 202) und
einem Strömungsprofil (42; 204),
das sich entlang einer Länge von einer proximalen Wurzel (44; 206) an der Plattform (46; 202) zu einem distalen Ende (48; 208) erstreckt und das
einen vorderen und einen hinteren Rand (60, 62; 210, 212) hat, die eine Druck- und eine Saugseite (64, 66; 214, 216) trennen, und mit
einem Kühlpassagennetzwerk, das wenigstens einen Hinterrandhohlraum (70) und mehrere Hinterrandöffnungen (80) aufweist, die sich vom hinteren Rand (62; 212) zum Hinterrandhohlraum (70) erstrecken, wobei das Verfahren einschließt:
Formen einer Turbinenelementvorform;
gleichzeitiges Ausbilden einer ersten Gruppe der mehreren Öffnungen (80) durch spanende Bearbeitung; und
gleichzeitiges Herstellen einer zweiten Gruppe der mehreren Öffnungen (80) durch spanende Bearbeitung, die entlang des hinteren Randes (62; 212) von der ersten Gruppe von Öffnungen versetzt sind und einen charakteristischen Abstand haben, der sich von dem charakteristischen Abstand der ersten Gruppe unterscheidet;
wobei Mittellinien (500) der ersten Gruppe von Öffnungen (80) nicht parallel zu Mittellinien der zweiten Gruppe von Öffnungen (80) sind.

2. Verfahren nach Anspruch 1, wobei der wenigstens eine Hinterrandhohlraum (70) ein Prallhohlraum ist.

3. Verfahren nach Anspruch 1 oder 2, wobei die Mittellinien der ersten und der zweiten Gruppe von Öffnungen (80) im Wesentlichen rechtwinklig zum hinteren Rand (62; 212) sind.

4. Verfahren nach einem der Ansprüche 1, 2 oder 3, wobei das Ausbilden der ersten Gruppe durch spanende Bearbeitung unter Benutzung einer ersten Vorrichtung (150) durchgeführt wird und das Ausbilden der zweiten Gruppe durch spanende Bearbeitung unter Benutzung einer zweiten Vorrichtung (150) durchgeführt wird und wobei das Verfahren zusätzlich einschließt, unter Benutzung der ersten Vorrichtung gleichzeitig eine dritte Gruppe der mehreren Hinterrandöffnungen (80) durch spanende Bearbeitung auszubilden.

5. Turbinenelement (40; 200) mit:
einem Strömungsprofil (42; 204), das ein erstes und ein zweites Ende (44, 48; 206, 208);
einem vorderen und einem hinteren Rand (60, 62; 210, 212); und
einem inneren Kühlpassagennetzwerk, das wenigstens einen Hinterrandhohlraum (70) und mehrere Hinterrandöffnungen (80) hat, die sich vom hinteren Rand (62; 212) zum Hinterrandhohlraum (70) erstrecken,
wobei die Hinterrandöffnungen in einem Abstand angeordnet sind, der sich entlang eines Bereiches des hinteren Randes (62; 212) vom ersten Ende (44; 206) zum zweiten Ende (48; 208) fortschreitend ändert;
**dadurch gekennzeichnet, dass** es verschiedene Gruppen von Hinterrandöffnungen (80) gibt, wobei die Öffnungen in jeder der Gruppe Mittellinien (500) haben, die parallel zueinander sind, und wobei die Mittellinien (500) der Öffnungen in wenigstens zwei der Gruppen nicht parallel zueinander sind.

6. Element nach Anspruch 5, wobei die Mittellinien (500) von Öffnungen in wenigstens einer der Gruppen im Wesentlichen rechtwinklig zum hinteren Rand sind.

7. Element nach Anspruch 5 oder 6, wobei der Abstand im Wesentlichen kontinuierlich zunimmt.

8. Element nach Anspruch 5 oder 6, wobei der Abstand im Wesentlichen schrittweise über Gruppen der Hinterrandöffnungen (80) zunimmt.

9. Element nach einem der Ansprüche 5 bis 8, das eine Laufschaufel (200) ist, wobei das zweite Ende eine freie Spitze (208) und das erste Ende ein Wurzelende (206) ist.

10. Element nach einem der Ansprüche 5 bis 8, das eine Leitschaufel (40) ist, wobei die ersten und zweiten Enden (44, 48) jeweils mit einer inneren und äußeren Plattform (46, 50) zusammenlaufen, die einheitlich mit dem Strömungsprofil (44) ausgebildet sind.

11. Element nach einem der Ansprüche 5 bis 10, wobei die Hinterrandöffnungen (80) Durchmesser zwischen 0.015 und 0.025 inch (0,381 und 0,635 mm) haben.

12. Element nach einem der Ansprüche 5 bis 11, wobei der Hinterrandhohlraum (70) ein Prallhohlraum ist.

13. Element nach einem der Ansprüche 5 bis 12, wobei:
das Netzwerk eingerichtet ist, Kühlgas innerhalb des Hinterrandhohlraums (70) zu führen, damit dessen Temperatur in einer ersten Richtung parallel zum hinteren Rand (62; 212) zunimmt; und
der Abstand in einer ersten Richtung im Wesentlichen fortschreitend abnimmt.

14. Element nach einem der Ansprüche 5 bis 13, wobei:
das Netzwerk eingerichtet ist, Kühlgas innerhalb des Hinterrandhohlraums (70) zu führen, damit dessen Temperatur in einer ersten Richtung parallel zum hinteren Rand (62; 212) zunimmt;
der Bereich wenigstens 50% einer Länge des hinteren Randes (62; 212) ist; und
der Abstand in einer ersten Richtung kontinuierlich fortschreitend abnimmt.

## Revendications

1. Procédé de fabrication d'un élément de turbine (40; 200), comprenant:
une plate-forme (46; 202); et
une aube (42; 204);
s'étendant le long d'une longueur à partir d'un pied proximal (44; 206) à la plate-forme (46; 202) jusqu'à une extrémité distale (48; 208);
comprenant des bords d'attaque et de fuite (60, 62; 210, 212) qui séparent les côtés de pression et d'aspiration (64, 66; 214, 216); et
comprenant un réseau de passages de refroidissement qui comprend au moins une cavité de bord de fuite (70) et une pluralité de trous de bord de fuite (80) qui s'étendent à partir du bord de fuite (62; 212) jusqu'à la cavité de bord de fuite (70),
le procédé comprenant les étapes suivantes:
couler un précurseur d'élément de turbine;
usiner simultanément les uns avec les autres un premier groupe de ladite pluralité desdits trous (80); et
usiner simultanément les uns avec les autres un deuxième groupe de ladite pluralité de trous (80), déplacé par rapport au premier groupe de trous le long du bord de fuite (62; 212), présentant un écartement caractéristique qui diffère d'un écartement caractéristique du premier groupe;
dans lequel les axes médians (500) dudit premier groupe de trous (80) sont non parallèles aux axes médians dudit deuxième groupe de trous (80).

2. Procédé selon la revendication 1, dans lequel ladite au moins une cavité de bord de fuite (70) est une cavité d'impact.

3. Procédé selon la revendication 1 ou 2, dans lequel les axes médians desdits premier et deuxième groupes de trous (80) sont sensiblement perpendiculaires au bord de fuite (62; 212).

4. Procédé selon la revendication 1, 2 ou 3, dans lequel l'usinage du premier groupe est exécuté en utilisant un premier dispositif (150), et l'usinage du deuxième groupe est exécuté en utilisant un deuxième dispositif (150), et le procédé comprend en outre l'usinage simultané d'un troisième groupe de ladite pluralité de trous de bord de fuite (80) en utilisant ledit premier dispositif.

5. Elément de turbine (40; 200), comprenant:
une aube (42; 204), comprenant:
des première et deuxième extrémités (44, 48; 206, 208);
des bords d'attaque et de fuite (60, 62; 210, 212); et
un réseau de passages de refroidissement internes comprenant au moins une cavité de bord de fuite (70) et une pluralité de trous de bord de fuite (80) qui s'étendent à partir du bord de fuite (62; 212) jusqu'à la cavité de bord de fuite (70),
dans lequel les trous de bord de fuite sont arrangés avec un écartement qui varie progressivement le long d'une partie dudit bord de fuite (62; 212) à partir de la première extrémité (44; 206) en direction de la deuxième extrémité (48; 208);
**caractérisé en ce que** plusieurs groupes desdits trous de bord de fuite (80) sont prévus, les trous dans chacun desdits groupes possédant des axes médians (500) parallèles les uns aux autres, et les axes médians (500) de trous dans au moins deux desdits groupes étant non parallèles les uns aux autres.

6. Elément selon la revendication 5, dans lequel les axes médians (500) de trous dans au moins un desdits groupes sont sensiblement perpendiculaires au bord de fuite.

7. Elément selon la revendication 5 ou 6, dans lequel l'écartement augmente de façon sensiblement continue.

8. Elément selon la revendication 5 ou 6, dans lequel l'écartement augmente sensiblement par étapes pour les groupes desdits trous de bord de fuite (80).

9. Elément selon l'une quelconque des revendications 5 à 8, ledit élément étant une pale (200) dans laquelle ladite deuxième extrémité est une pointe libre (208), et la première extrémité est une extrémité de pied (206).

10. Elément selon l'une quelconque des revendications 5 à 8, ledit élément étant une ailette (40) dans laquelle lesdites première et deuxième extrémités (44, 48) joignent respectivement les plates-formes intérieure et extérieure (46, 50) qui sont formées de façon unitaire avec l'aube (44).

11. Elément selon l'une quelconque des revendications 5 à 10, dans lequel les trous de bord de fuite (80) ont des diamètres qui sont compris entre 0.015 pouce et 0.025 pouce (0,381 mm et 0,635 mm).

12. Elément selon l'une quelconque des revendications 5 à 11, dans lequel la cavité de bord de fuite (70) est une cavité d'impact.

13. Elément selon l'une quelconque des revendications 5 à 12, dans lequel:
le réseau est adapté pour diriger un gaz de refroidissement à l'intérieur de la cavité de bord de fuite (70) afin d'augmenter la température dans une première direction parallèle au bord de fuite (62; 212); et
l'écartement diminue de façon sensiblement progressive dans la première direction.

14. Elément selon l'une quelconque des revendications 5 à 13, dans lequel:
le réseau est adapté pour diriger un gaz de refroidissement à l'intérieur de la cavité de bord de fuite (70) afin d'augmenter la température dans une première direction parallèle au bord de fuite (62; 212);
la partie correspond à au moins 50 % d'une longueur du bord de fuite (62; 212); et
l'écartement diminue de façon progressivement continue dans la première direction.
